# EUROPEAN PATENT SPECIFICATION

(11) **EP 1 685 849 B1**
(45) Date of publication and mention of the grant of the patent: **25.01.2012**
(21) Application number: 04799810.9
(22) Date of filing: 18.11.2004
(51) Int. Cl.: A61K 45/00, A61K 31/5377, A61K 31/4409, A61K 31/4375, A61K 31/277, C07D 213/81, C07D 213/75, C07D 471/04, A61P 29/00, A61P 13/10

(54) **PDE 4 INHIBITORS FOR THE TREATMENT OF INTERSTITIAL CYSTITIS**
PDE 4 INHIBITOREN ZUR BEHANDLUNG DER INTERSTITIELLEN CYSTITIS
INHIBITEURS DE PHOSPHODIESTERASE 4 (PDE 4) POUR LE TRAITEMENT DE LA CYSTITE INTERSTITIELLE

(30) Priority: 20.11.2003 JP 2003390137
(43) Date of publication of application: 02.08.2006
(73) Proprietor: Astellas Pharma Inc., Tokyo 103-8411 (JP)
(72) Inventor: KOBAYASHI, Miki, c/o Astellas Pharma Inc.,, Chuo-ku, Tokyo 103-8411 (JP); KUBO, Satoshi, c/o Astellas Pharma Inc., Chuo-ku, Tokyo 103-8411 (JP)
(74) Representative: HOFFMANN EITLE
(86) International application number: PCT/JP2004/017521
(87) International publication number: WO 2005/049087

(56) References cited:
- JP-A- 2002 513 914
- HATZELMANN, A. ET AL.: 'Anti-inflammatory and immunomodulatory potential of the novel PDE4 inhibitor roflumilast in vitro' J PHARMACOL EXP THER. vol. 297, no. 1, April 2001, pages 267 - 279, XP001024814

## Description

### Technical Field

This invention relates to an agent for preventing/treating interstitial cystitis.

### Background of the Invention

Interstitial cystitis is a non-infectious inflammatory disease having urinary urgency and frequent urination or pain in the bladder and the periphery of the pelvis as its symptoms (Non-patent References 1 and 2), and it has been reported that there are 450,000 to 1,000,000 patients of this disease in the United Stats, and 90% of them are females. Though its definite cause is not clear yet, increase of mast cell, eosinophil and T cell is found in the bladder tissue of the patients (Non-patent References 3 to 6), and markers which indicate epithelial injury, activation of mast cell and eosinophil infiltration and inflammatory cytokine are increased in the urine (Non-patent Reference 7). In addition, since deposition of immunoglobulin and complement onto the bladder tissue is identified, and a case of accompanying complication of diseases such as bronchial asthma and articular rheumatism is frequently found, involvement of systemic allergy and autoimmune disease has been suggested (Non-patent References 8 to 10). Based on these, a possibility can be considered as a phase of the disease state of interstitial cystitis that chronic inflammation caused by the activation of inflammatory cells including mast cell and overexpression of various growth factors and cytokines accompanied thereby induce excessive repair and fibrosis of the tissue and hypersensitivity of sensory nerves, and these are reflected in the frequent urination and pain in the bladder and the periphery of the pelvis.

However, steroid drugs which have broad antiinflammatory activities and have significant effects upon asthma and atopic dermatitis are hardly used for interstitial cystitis. Though a bladder hydrodistension, an intravesical administration therapy (dimethyl sulfoxide (DMSO) or heparin), oral medicines (antidepressant, antihistaminic, non-steroidal antiinflammatory drugs (NSAIDs) and the like) can be exemplified as other therapeutic method, it is the present situation that there is no unified therapeutic method and effective preventive or therapeutic agent which could become a standard (Non-patent References 11, 12 and 13).

By the way, cyclic nucleotide phosphodiesterase (PDE) is an enzyme which metabolizes intracellular signal transmitters cyclic adenosine monophosphate (cAMP) and cyclic guanosine monophosphate (cGMP) into 5'-adenosine monophosphate (5'-AMP) and 5'-guanosine monophosphate (5'-GMP), respectively, and play an important role in the regulation of intracellular cAMP and cGMP concentrations. The presence of at least 11 species of isozymes in PDE has so far been reported (Non-patent References 19 and 20), and since their tissue distribution differs by each isozyme, there is a possibility that principal isozymes which metabolize cAMP or cGMP are different by tissues (Non-patent Reference 21).

PDE 4 is one of the PDE isozymes, which is expressed in inflammatory cells such as leukocyte.

Since, as a result of pharmacological studies so far carried out, its inhibitors markedly inhibit activation of immune and inflammatory cells and exert broad antiinflammatory actions (Non-patent References 22 and 23), it has been reported that they are effective for asthma, chronic obstructive pulmonary disease, articular rheumatism, multiple sclerosis and the like. However, these references do not report on the efficacy and usability of PDE 4 inhibitors as preventive or therapeutic agents for chronic pelvic pain syndrome such as interstitial cystitis.

As the PDE 4 inhibitors, various compounds have been reported (e.g., see Patent References 6 to 10), including roflumilast (e.g., Non-patent Reference 24, Patent Reference 1), cilomilast (e.g., Non-patent Reference 25, Patent References 2 and 3), 3-[4-(3-chlorophenyl)-1-ethyl-7-methyl-2-oxo-1,2-dihydro-1,8-naphthyridin-3-yl]propanoic acid (Patent Reference 4) and 4-(4-{4-[6-(3,4-dimethoxyphenyl)pyridine-2-carbonyl]piperazin-1-yl}phenyl)morpholine (Patent Reference 5). However, these references also do not report on the efficacy and usability of PDE 4 inhibitors as preventive or therapeutic agents for chronic pelvic pain syndrome such as interstitial cystitis. Patent reference 11 discloses a method of diagnosing Chronic Pelvic Pain Syndrome in men comprising measuring levels of cytokines in semen or components or fractions of semen. It also discloses a method of treating a condition associated with elevated levels of a cytokine, such as TNF- alpha, in semen or a component or fraction thereof, comprising administering a therapeutically effective amount of an anti-cytokine compound or composition, such as an anti-TNF- alpha compound or composition.

[Non-patent Reference 1] Gillenwater J.Y., Wein A.J., "Summary of the National Institute of Arthritis, Diabetes, Digestive and Kidney Diseases Workshop on Interstitial Cystitis", National Institutes of Heals, Bethesda, Maryland, (USA), August 1987, pp. 28 - 29

[Non-patent Reference 2] The Journal of Urology, (USA), July 1988, vol. 140, no. 1, pp. 203 - 6

[Non-patent Reference 3] British Journal of Urology, (England), June 1982, vol. 54, no. 3, pp. 283 - 6

[Non-patent Reference 4] British Journal of Urology, (England), October 1983, vol. 55, no. 5, pp. 495 - 500

[Non-patent Reference 5] International Journal of Urology, (Australia), July 1998, vol. 5, no. 4, pp. 329 - 35

[Non-patent Reference 6] The Journal of Urology, (USA), September 1997, vol. 158, no. 3, part 1, pp. 790 - 3

[Non-patent Reference 7] Urology, (USA), July 2001, vol. 57, no. 6 supplement 1, pp. 15 - 21

[Non-patent Reference 8] American Journal of Clinical Pathology, (USA), November 1979, vol. 72, no. 5, pp. 777 - 84

[Non-patent Reference 9] The Journal of Urology, (USA), March 1993, vol. 149, no. 3, pp. 465 - 9

[Non-patent Reference 10] Urology, (USA), May 1997, vol. 49, no. 5, supplement 1, pp. 52 - 7

[Non-patent Reference 11] "Kanshitsusei Bokoen - Ekigaku Kara Chiryo Made - (Interstitial Cystitis - From Epidemiology To Treatment)", edited by Japanese Society for the Research of Interstitial Cystitis, published by Igaku Tosho Shuppan on April 20, 2002

[Non-patent Reference 12] Urology, (USA), December 20, 2000, vol. 56, no. 6, pp. 940 - 5

[Non-patent Reference 13] Expert Opinion on Investigational Drugs, (England), 2001, March, vol. 10, no. 3, pp. 521 - 46

[Non-patent Reference 19] American Journal of Respiratory and Critical Care Medicine, (USA), 1998, vol. 157, pp. 351 - 370

[Non-patent Reference 20] Current Opinion in Cell Biology, (USA), 2000, vol. 12, pp. 174 - 179

[Non-patent Reference 21] The Journal of Allergy and Clinical Immunology, (USA), 2001, vol. 108, pp. 671 - 680 [Non-patent Reference 22] Trends in Pharmacological Sciences, (England), 1997, vol. 18, pp. 164 - 171

[Non-patent Reference 23] Immunopharmacology, (Holland), 2000, vol. 47, pp. 127 - 162

[Non-patent Reference 24] The Journal of Pharmacology and Experimental Therapeutics, (USA), April 2001, vol. 297, no. 1, pp. 267 - 79

[Non-patent Reference 25] The Journal of Pharmacology and Experimental Therapeutics, (USA), December 1998, vol. 287, no. 3, pp. 988 - 95

[Patent reference 1] International Publication 95/01338

[Patent reference 2] International Publication 95/24381

[Patent reference 3] International Publication 01/87281

[Patent reference 4] International Publication 01/30779

[Patent reference 5] International Publication 02/102778

[Patent reference 6] International Publication 96/06843

[Patent reference 7] International Publication 97/19078

[Patent reference 8] JP-A-11-292878

[Patent reference 9] JP-A-11-292877

[Patent reference 10] US Patent 6544983

[Patent reference 11] International Publication 99/57303

### Disclosure of the Invention

As described in the above, a unified therapeutic method for interstitial cystitis has not been established, and there is no drug effective as its preventive or treating agent, so that great concern has been directed toward the establishment of a standard treating method and development of effective preventive or treating agent.

Under such a situation, the present inventors have carried out intensive studies with the aim of developing a preventive or therapeutic agent for interstitial cystitis in which sufficient preventive or therapeutic effect has not been obtained by the existing antidepressant, antihistaminic, NSAIDs or steroid agents. As a result, it was found unexpectedly that PDE 4 inhibitors, 3-[4-(3-chlorophenyl)-1-ethyl-7-methyl-2-oxo-1,2-dihydro-1,8-naphthyridin-3-yl]propanoic acid, roflumilast and cilomilast, inhibit infiltration of granulocyte into the bladder in a test method using a rat antigen-induced cystitis model, and it was found a possibility that PDE 4 inhibitors represented by these compounds are useful as agents for treating interstitial cystitis. As a result of further conducting intensive studies, it was found that a PDE 4 inhibitor 3-[4-(3-chlorophenyl)-1-ethyl-7-methyl-2-oxo-1,2-dihydro-1,8-naphthyridin-3-yl]propanoic acid has a therapeutic effect upon considerably damaged micturition reflex and bladder function, thus accomplishing the present invention.

That is, the present invention relates to
(1) a pharmaceutical preparation for preventing or treating interstitial cystitis
   , which comprises a phosphodiesterase 4 (PDE 4) inhibitor as an active ingredient,
(2) the pharmaceutical preparation described in (1), wherein the PDE 4 inhibitor is a compound selected from the group consisting of roflumilast, cilomilast, 3-[4-(3-chlorophenyl)-1-ethyl-7-methyl-2-oxo-1,2-dihydro-1,8-naphthyridin-3-yl]propanoic acid, 4-(4-{4-[6-(3,4-dimethoxyphenyl)pyridine-2-carbonyl]piperazin-1-yl}phenyl)morpholine, and salts thereof,
(3) use of a PDE 4 inhibitor for the manufacture of a pharmaceutical preparation for preventing or treating interstitial cystitis syndrome,
(4) the use described in (3),
   wherein the PDE 4 inhibitor is a compound selected from the group consisting of roflumilast, cilomilast, 3-[4-(3-chlorophenyl)-1-ethyl-7-methyl-2-oxo-1,2-dihydro-1,8-naphthyridin-3-yl]propanoic acid, 4-(4-{4-[6-(3,4-dimethoxyphenyl)pyridine-2-carbonyl]piperazin-1-yl}phenyl)morpholine, and salts thereof.

The following describes the present invention in detail.

According to this specification, the "PDE 4 inhibitor" is not particularly limited, with the proviso that it is a compound which has the PDE 4 inhibitory activity and has a preventing or treating effect upon the "chronic pelvic pain syndrome" defined by the present invention, and, for example, a compound having an IC₅₀ value of 5 µM or less, particularly an IC₅₀ value of 500 nM or less, measured by the PDE 4 inhibitory activity test described in Test Example 1 of International Patent Publication 01/30779, may be exemplified. As such a PDE 4 inhibitor, roflumilast, cilomilast, 3-[4-(3-chlorophenyl)-1-ethyl-7-methyl-2-oxo-1,2-dihydro-1,8-naphthyridin-3-yl]propanoic acid (to be referred to as compound A hereinafter), 4-(4-{4-[6-(3,4-dimethoxyphenyl)pyridine-2-carbonyl]piperazin-1-yl}phenyl)morpholine, pharmaceutically acceptable salts thereof, and the PDE 4 inhibitors disclosed in the aforementioned Patent References 6 to 10 and the like may be illustratively exemplified. Preferred among them are roflumilast, cilomilast, 3-[4-(3-chlorophenyl)-1-ethyl-7-methyl-2-oxo-1,2-dihydro-1,8-naphthyridin-3-yl]propanoic acid, 4-(4-{4-[6-(3,4-dimethoxyphenyl)pyridine-2-carbonyl]piperazin-1-yl}phenyl)morpholine and pharmaceutically acceptable salts thereof. These PDE 4 inhibitors may be easily obtained by the production methods described in said patent references. In addition, the present invention includes a pharmaceutical preparation for preventing or treating , which jointly uses one or two or more of these PDE 4 inhibitors.

The aforementioned PDE 4 inhibitors may also form acid addition salts or salts with bases in some cases, and such salts are included in the invention with the proviso that they are pharmaceutically acceptable salts. Illustratively, an acid addition salt with inorganic acid such as hydrochloric acid, hydrobromic acid, hydroiodic acid, sulfuric acid, nitric acid, and phosphoric acid or with organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, and glutamic acid, a salt with inorganic base such as sodium, potassium, magnesium, calcium, and aluminum or with organic base such as methylamine, ethylamine, ethanolamine, lysine, and ornithine, and an ammonium salt and the like can be exemplified. In addition, the PDE 4 inhibitors or pharmaceutically acceptable salts thereof may be in the form of various hydrates or solvates, and their polymorphic substances are further included. Salts of these PDE 4 inhibitors can be easily produced by salt forming methods which can be generally employed by those skilled in the art.

Also, mixtures and isolated forms of various stereoisomers such as geometrical isomers, tautomers, and optical isomers are included in the aforementioned PDE 4 inhibitors. These isomers can be isolated and purified by conventionally known methods such as extraction, precipitation, differential chromatography, fractional crystallization, recrystallization and the like. In addition, optical isomers can be resolved by usual methods such as a fractional crystallization in which they are recrystallized with an appropriate salt, a column chromatography and the like.

The pharmaceutical preparation which comprises a PDE 4 inhibitor as an active ingredient is prepared using carriers, fillers and other additive agents generally used in preparing pharmaceutical preparations.

Its administration may be either oral administration by tablets, pills, capsules, granules, powders, solutions or the like or parenteral administration by injections for intravenous injection, intramuscular injection or the like, suppositories, percutaneous preparations, transnasal preparations, inhalations, intravesical injections or the like. The dose is optionally decided by taking into consideration symptoms, age, sex and the like of each patient to be administered, but is usually approximately from 0.001 mg/kg to 100 mg/kg per adult per day in the case of oral administration, and this is administered once or dividing into 2 to 4 times. Also, when intravenously administered due to the symptom, this is administered generally within the range of from 0.0001 mg/kg to 10 mg/kg per adult per once, once a day or two or more times a day. In addition, in the case of inhalation, this is administered generally within the range of from 0.0001 mg/kg to 1 mg/kg per adult per once, once a day or two or more times a day.

As the solid composition for use in the oral administration according to the present invention, tablets, powders, granules and the like are used. In such a solid composition, one or more active substances are mixed with at least one inert filler such as lactose, mannitol, glucose, hydroxypropylcellulose, microcrystalline cellulose, starch, polyvinyl pyrrolidone, aluminum magnesium silicate or the like. In the usual way, the composition may contain inert additives such as lubricant such as magnesium stearate, disintegrating agent such as carboxymethylstarch sodium, a solubilizing and a solubilization assisting agent. As occasion demands, tablets or pills may be coated with a sugar coating or a film of a gastric or enteric coating agent.

The liquid composition for oral administration includes pharmaceutically acceptable emulsions, solutions, suspensions, syrups, elixirs and the like and contains a generally used inert solvent such as purified water or ethanol. In addition to the inert solvent, this composition may also contain auxiliary agents such as a solubilizing agent, a moistening agent, and a suspending agent, as well as sweeteners, flavors, aromatics and antiseptics.

The injections for parenteral administration includes aseptic aqueous or non-aqueous solutions, suspensions and emulsions. Examples of the aqueous solvent include distilled water for injection and physiological saline. Examples of the non-aqueous solvent include propylene glycol, polyethylene glycol, plant oil such as olive oil, alcohol such as ethyl alcohol, polysorbate 80 (trade name) and the like. Such a composition may further contain a tonicity agent, an antiseptic, a moistening agent, an emulsifying agent, a dispersing agent, a stabilizing agent, a solubilizing agent and solubilization assisting agent. These compositions are sterilized for example by filtration through a bacteria retaining filter, blending of a germicide or irradiation. Alternatively, they may be used by firstly making into sterile solid compositions and dissolving or suspending them in sterile water or a sterile solvent for injection prior to their use.

Transmucosal preparations such as inhalations and transnasal preparations are used in the solid, liquid or semisolid form and can be produced in accordance with the conventionally known methods. For example, filler such as lactose and starch, as well as a pH adjusting agent, an antiseptic, a surfactant, a lubricant, a stabilizer, a thickener and the like may be optionally added. An appropriate device for inhalation or blowing can be used for the administration. For example, a compound can be administered as it is, or as a powder of a formulated mixture or as a solution or suspension by combining with a pharmaceutically acceptable carrier, using conventionally known device or a nebulizer such as a measured administration inhalation device. A dry powder inhaler or the like may be for single or multiple time administration use, and dry powders or powder-containing capsules can be used. Alternatively, it may be a pressure aerosol spray or the like form which uses a suitable gas of appropriate propellant such as chlorofluoroalkane, hydrofluoroalkane, carbon dioxide or the like.

### Brief Description of the Drawings

Fig. 1 shows a result of the 3-[4-(3-chlorophenyl)-1-ethyl-7-methyl-2-oxo-1,2-dihydro-1,8-naphthyridin-3-yl]propanoic acid (compound A) administered group in Example 1.
Fig. 2 shows a result of the roflumilast administered group and cilomilast administered group in Example 1.
Fig. 3 shows a result of the compound A administered group in Example 2.

### Best Mode for Carrying Out the Invention

The following illustratively describes the present invention based on examples, but these do not limit the scope of the invention.

### Example 1

### Efficacy of PDE 4 inhibitors in rat antigen-induced cystitis model

Antigen sensitization was carried out by intraperitoneally administering physiological saline containing ovalbumin (OA, 1 mg/ml)-aluminum hydroxide gel (Alum, 20 mg/ml) to Brown Norway (BN) female rats, at a dose of 1 ml per animal for 3 continuous days. After completion of the sensitization, a urethral catheter was attached to each animal under anesthesia, 3% OA/physiological saline was injected into the bladder to induce cystitis, and the resulting animals were used as the control group. Also, a group in which physiological saline was injected into the bladder of each sensitized rat was used as the physiological saline group. A group in which compound A (3 mg/kg), roflumilast (3 mg/kg) or cilomilast (30 mg/kg) was orally administered 1 hour before the intravesical antigen injection was used as a compound A administration group, a roflumilast administration group or a cilomilast administration group. After 24 hours of the intravesical antigen injection, the bladder was removed under pentobarbital anesthesia, and bladder extract was prepared in accordance with the method of Malley et al. (Physiological Genomics, (USA), 2002, vol. 9, no. 1, pp. 5 - 13). Peroxidase activity in the bladder extract was measured in accordance with the method of Bradley et al. (The Journal of Investigative Dermatology, (England), March 1982, vol. 78, no. 3, pp. 206 - 9) using human myeloperoxidase (manufactured by Sigma) as the standard, and the results were expressed by defining the peroxidase activity of the physiological saline group as 0%, and the peroxidase activity of the control group as 100%.

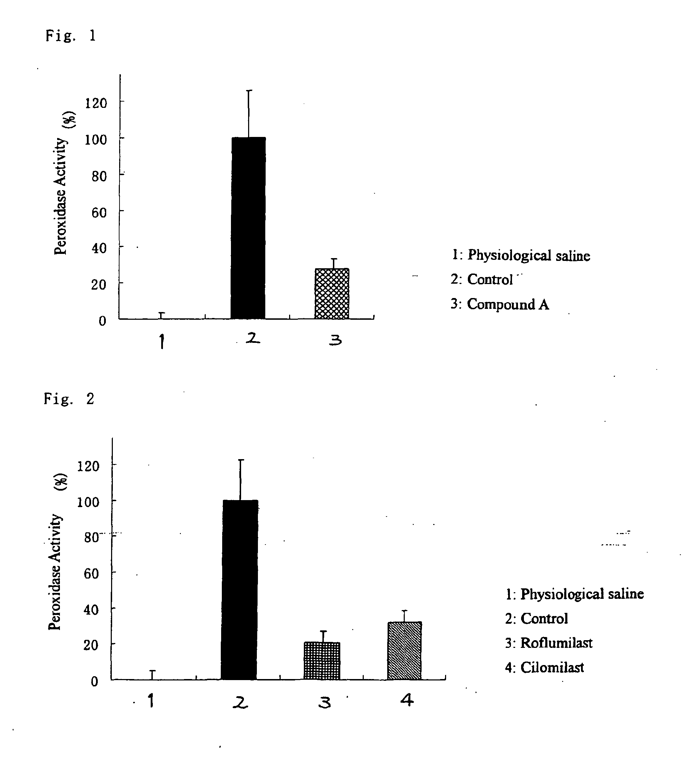

When antigen-induced cystitis was caused by carrying out antigen injection into the bladder of each of the sensitized BN rats, and peroxidase activity in the bladder tissue was measured as one of the indexes of cystitis, increase in the peroxidase activity in the bladder tissue was found in the control group. In this connection, the presence of eosinophil peroxidase in this increased peroxidase activity was confirmed by 3-amino-1,2,4-triazole (5 mM) (Journal of Immunological Methods, (Holland), May 11, 1984, vol. 70, no. 1, pp. 119 - 25). In the roflumilast administration group, the cilomilast administration group and the compound A administration group, in which respective PDE 4 inhibitors were administered, the increase of peroxidase activity was inhibited 79%, 68% and 72%, respectively (Figs. 1 and 2). That is, infiltration of granulocytes including eosinophil into the antigen induced bladder was inhibited.

Since it is considered that a chronic inflammation caused by the activation of inflammatory cells and overexpression of various growth factors and cytokine accompanied thereby are reflected in the disease state of interstitial cystitis, and these PDE 4 inhibitors inhibited infiltration of granulocytes into the antigen induced bladder, there is a possibility that PDE 4 inhibitors are useful as therapeutic agents for interstitial cystitis.

### Example 2

### Efficacy of PDE 4 inhibitors in rat hydrochloric acid-induced cystitis model

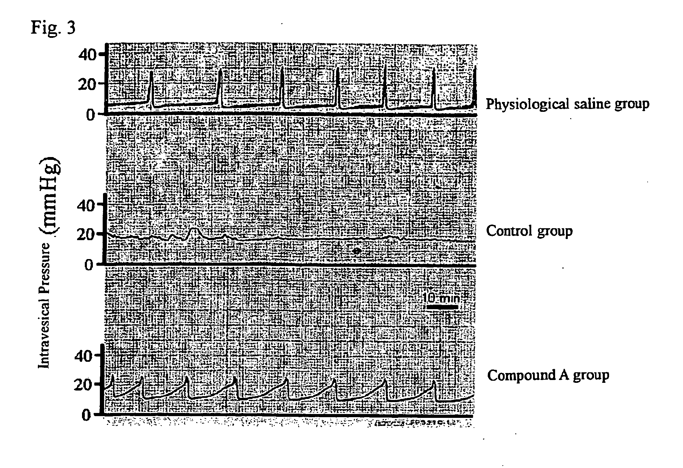

A bladder disorder was induced by attaching a urethral catheter to each of Brown Norway (BN) female rats under ether anesthesia and injecting 0.4 N hydrochloric acid into the bladder (0.15 ml per one rat). Starting on the next of the bladder disorder induction, the compound A (1 mg/kg) or a vehicle (0.5% methyl cellulose aqueous solution) was orally administered once a day for 10 days, and respectively used as a compound A administration group and a control group. Also, a group in which physiological saline was injected into the bladder (0.15 ml per one rat), and the vehicle was orally administered once a day for 10 days starting on the next day, was used as a physiological saline group. After 11 days of the bladder disorder induction, each rat was fixed in the supine position under urethane anesthesia and equipped with a urethral catheter, and via its three-way cock, one end was used as the injection passage and the other end was connected to a pressure transducer (DX-312, manufactured by NIHON KOHDEN). Physiological saline was continuously injected into the bladder (50 µl/min) using an infusion pump (model 22, Harvard), and the intravesical pressure at that time was amplified by a pre-amplifier (AP-621G, manufactured by NIHON KOHDEN) and recorded using a recorder. A typical intravesical pressure pattern is shown in Fig. 3. Micturition reflex of almost constant interval was observed in the physiological saline group, and the resting time intravesical pressure was 10 mmHg or less, and the maximum bladder contraction pressure at the time of micturition was about 30 mmHg. In comparison with this, in the case of the control group, increase in the resting time intravesical pressure was observed, but increase in the bladder contraction pressure at the time of micturition was hardly found, and micturition frequency was high. In the compound A administration group, increase of the resting time intravesical pressure was inhibited, and increase in the intravesical pressure accompanied by the micturition reflex was also observed. In addition, interval of the micturition reflex was also almost the same as that of the physiological saline administration group. Based on these results, it was shown that the compound A as a PDE 4 inhibitor is possessed of a therapeutic effect upon the micturition reflex and bladder function markedly damaged by the injection of hydrochloric acid into the bladder.

### Industrial Applicability

As described in the above, it was shown that PDE 4 inhibitors including the compound A and roflumilast are possessed of the therapeutic effect in the test methods of interstitial cystitis. Thus, according to the invention, it rendered possible the provision of a medicament which comprises a phosphodiesterase 4 (PDE 4) inhibitor as an active ingredient, as a preventive or therapeutic agent for interstitial cystitis which has been lacking in effective preventive or therapeutic agents.

## Claims

1. A pharmaceutical preparation for use in preventing or treating interstitial cystitis, which comprises a phosphodiesterase 4 (PDE 4) inhibitor as an active ingredient.

2. The pharmaceutical preparation for use in preventing or treating interstitial cystitis described in Claim 1,
wherein the PDE 4 inhibitor is a compound selected from the group consisting of roflumilast, cilomilast, 3-[4-(3-chlorophenyl)-1-ethyl-7-methyl-2-oxo-1,2-dihydro-1,8-naphthyridrin-3-yl]propanoic acid, 4-(4-{4-[6-(3,4-dimethoxyphenyl)pyridine-2-carbonyl]piperazin-1-yl}phenyl)morpholine, and salts thereof.

3. Use of a PDE 4 inhibitor for the manufacture of pharmaceutical preparation for preventing or treating interstitial cystitis.

4. The use described in Claim 3, wherein the PDE 4 inhibitor is a compound selected from the group consisting of roflumilast, cilomilast, 3-[4-(3-chlorophenyl)-1-ethyl-7-methyl-2-oxo-1,2-dihydro-1,8-naphthyridrin-3-yl]-propanoic acid, 4-(4-{4-[6-(3,4-dimethoxyphenyl)pyridine-2-carbonyl]piperazin-1-yl}phenyl)morpholine, and salts thereof.

## Patentansprüche

1. Pharmazeutische Zubereitung zur Verwendung bei der Vorbeugung oder Behandlung von interstitieller Cystitis, die einen Phosphodiesterase 4 (PDE 4)-Inhibitor als wirksamen Bestandteil umfasst.

2. Pharmazeutische Zubereitung zur Verwendung bei der Vorbeugung oder Behandlung von interstitieller Cystitis gemäß Anspruch 1, worin der PDE 4-Inhibitor eine Verbindung ist, die aus Roflumilast, Cilomilast, 3-[4-(3-Chlorophenyl)-1-ethyl-7-methyl-2-oxo-1,2-dihydro-1,8-naphthyridin-3-yl]propansäure, 4-(4-{4-[6-(3,4-Dimethoxyphenyl)pyridin-2-carbonyl]piperazin-1-yl}phenyl)morpholin und Salzen daraus ausgewählt ist.

3. Verwendung eines PDE 4-Inhibitors zur Herstellung einer pharmazeutischen Zubereitung zur Vorbeugung oder Behandlung von interstitieller Cystitis.

4. Verwendung gemäß Anspruch 3, worin der PDE 4-Inhibitor eine Verbindung ist, die aus Roflumilast, Cilomilast, 3-[4-(3-Chlorophenyl)-1-ethyl-7-methyl-2-oxo-1,2-dihydro-1,8-naphthyridin-3-yl]propansäure, 4-(4-{4-[6-(3,4-Dimethoxyphenyl)pyridin-2-carbonyl]piperazin-1-yl}phenyl)morpholin und Salzen daraus ausgewählt ist.

## Revendications

1. Préparation pharmaceutique pour une utilisation dans la prévention ou le traitement d'une cystite interstitielle, qui comprend un inhibiteur de phospho-diestérase 4 (PDE 4) en tant qu'ingrédient actif.

2. Préparation pharmaceutique pour une utilisation dans la prévention ou le traitement d'une cystite interstitielle selon la revendication 1, dans laquelle l'inhibiteur de PDE 4 est un composé choisi dans le groupe constitué par le roflumilast, le cilomilast, l'acide 3-[4-(3-chlorophényl)-1-éthyl-7-méthyl-2-oxo-1,2-dihydro-1,8-naphtyridin-3-yl]propanoïque, la 4-(4-{4-[6-(3,4-diméthoxyphényl)pyridine-2-carbonyl]pipérazin-1-yl}phényl)morpholine, et leurs sels.

3. Utilisation d'un inhibiteur de PDE 4 pour la fabrication d'une préparation pharmaceutique pour prévenir ou traiter une cystite interstitielle.

4. Utilisation selon la revendication 3, dans laquelle l'inhibiteur de PDE 4 est un composé choisi dans le groupe constitué par le roflumilast, le cilomilast, l'acide 3-[4-(3-chlorophényl)-1-éthyl-7-méthyl-2-oxo-1,2-dihydro-1,8-naphtyridin-3-yl]propanoïque, la 4-(4-{4-[6-(3,4-diméthoxyphényl)pyridine-2-carbonyl]pipérazin-1-yl}phényl)morpholine, et leurs sels.
